# EUROPEAN PATENT APPLICATION

(11) **EP 4 582 519 A1**
(43) Date of publication of application: **09.07.2025**
(21) Application number: 23860436.7
(22) Date of filing: 30.08.2023
(51) Int. Cl.: C10G 2/00, C07C 29/151, C07C 31/04, C10L 1/02

(54) **METHOD FOR PRODUCING LIQUID FUEL AND LIQUID FUEL SYNTHESIS SYSTEM**

(30) Priority: 01.09.2022 JP 2022139588
(71) Applicant: NGK Insulators, Ltd., Nagoya-shi, Aichi 467-8530 (JP)
(72) Inventor: IIDA, Kazuki, Nagoya-shi, Aichi 467-8530 (JP); MAEHARA, Sota, Nagoya-shi, Aichi 467-8530 (JP); ANDO, Junichi, Nagoya-shi, Aichi 467-8530 (JP); OKUMA, Yusuke, Nagoya-shi, Aichi 467-8530 (JP); KAN, Hirofumi, Nagoya-shi, Aichi 467-8530 (JP); SHIBAGAKI, Yukinari, Nagoya-shi, Aichi 467-8530 (JP); TAKAHASHI, Michio, Nagoya-shi, Aichi 467-8530 (JP)
(74) Representative: Müller-Boré & Partner Patentanwälte PartG mbB
(86) International application number: PCT/JP2023/031602
(87) International publication number: WO 2024/048674

(57) **Abstract**

A primary object of the present invention is to reduce a temperature difference between a gas temperature at the inlet of a reactor and a gas temperature at the outlet thereof in a conversion reaction from a raw material gas containing a carbon oxide and hydrogen to a liquid fuel. A method of producing a liquid fuel according to an embodiment of the present invention includes: causing a raw material gas containing at least a carbon oxide and hydrogen to flow into a reactor storing a catalyst; and producing the liquid fuel from the raw material gas through a conversion reaction in the presence of the catalyst. The raw material gas further contains an inert gas. A ratio of a concentration of carbon monoxide to a concentration of carbon dioxide in the carbon oxide is 0.9 or less.

## Description

### Technical Field

The present invention relates to a method of producing a liquid fuel and a liquid fuel synthesis system.

### Background Art

In recent years, the following has been proposed for the purpose of achieving a carbon neutral society: a carbon oxide, such as carbon monoxide or carbon dioxide, is grasped as a carbon resource, and is converted into a liquid fuel useful as an industrial basic raw material. In, for example, Patent Literature 1, there is a disclosure of a liquid fuel synthesis system that performs a conversion reaction from a raw material gas containing carbon dioxide (CO₂) and hydrogen (H₂) to methanol with a membrane reactor including a catalyst and a water vapor separation membrane.

### Citation List

### Patent Literature

[PTL 1] JP 2018-8940 A

### Summary of Invention

### Technical Problem

In such conversion reaction from a raw material gas containing a carbon oxide and hydrogen to a liquid fuel as described above, there has been a demand for a reduction in temperature difference between a gas temperature at the inlet of a reactor and a gas temperature at the outlet thereof.

A primary object of the present invention is to reduce a temperature difference between a gas temperature at the inlet of a reactor and a gas temperature at the outlet thereof in a conversion reaction from a raw material gas containing a carbon oxide and hydrogen to a liquid fuel.

### Solution to Problem

[1] According to one embodiment of the present invention, there is provided a method of producing a liquid fuel, including: causing a raw material gas containing at least a carbon oxide and hydrogen to flow into a reactor storing a catalyst; and producing the liquid fuel from the raw material gas through a conversion reaction in the presence of the catalyst, wherein the raw material gas further contains an inert gas, and wherein a ratio of a concentration of carbon monoxide to a concentration of carbon dioxide in the carbon oxide is 0.9 or less.
[2] In the production method according to the above-mentioned item [1], a concentration of the inert gas in the raw material gas may be 1 vol% or more and 55 vol% or less.
[3] In the production method according to the above-mentioned item [1] or [2], a ratio of a concentration of the inert gas to a concentration of the carbon dioxide in the raw material gas may be 0.05 or more and 1.5 or less.
[4] In the production method according to any one of the above-mentioned items [1] to [3], a concentration of the carbon oxide in the raw material gas may be 5 vol% or more and 50 vol% or less.
[5] In the production method according to any one of the above-mentioned items [1] to [4], a concentration of the hydrogen in the raw material gas may be 20 vol% or more and 85 vol% or less.
[6] In the production method according to any one of the above-mentioned items [1] to [5], the raw material gas may have a temperature of 40°C or more and 350°C or less when caused to flow into the reactor.
[7] The production method according to any one of the above-mentioned items [1] to [6] may further include preparing the raw material gas by using a gas containing an inert gas and carbon dioxide captured from air or a biogas.
[8] In the production method according to any one of the above-mentioned items [1] to [7], the reactor may include a water vapor separation membrane configured to cause water vapor to permeate therethrough.
[9] In the production method according to any one of the above-mentioned items [1] to [7], the reactor may include a liquid fuel separation membrane configured to cause at least the liquid fuel to permeate therethrough.
[10] According to another embodiment of the present invention, there is provided a method of producing a liquid fuel, including: causing a raw material gas containing at least a carbon oxide and hydrogen to flow into a reactor storing a catalyst; and producing the liquid fuel from the raw material gas through a conversion reaction in the presence of the catalyst, wherein the raw material gas further contains an inert gas, wherein the liquid fuel contains methanol, wherein an approximate straight line produced by subjecting points plotted on the basis of results of thermodynamic equilibrium calculation, which is performed on the conversion reaction at 200°C and 4 MPa (G) when a ratio of a concentration of carbon monoxide (CO) to a concentration of carbon dioxide (CO₂) in the carbon oxide in the raw material gas and a ratio of a concentration of the inert gas (Inert) to a concentration of the carbon dioxide (CO₂) in the raw material gas are changed, against an X-axis indicating a ratio " (Inert+CO)/CO₂" in the raw material gas and a Y-axis indicating a product " (ratio of suppressed heating value) × (ratio of obtained combustion energy increase)" to linear approximation (Y=aX+b) has a gradient "a" of 1.4 or less, wherein the plotting is performed on a total of 18 pieces of data when an Inert/CO₂ ratio is changed in a range of from 0 to 1.6 in increments of 0.1, and when the Inert/CO₂ ratio is 0.05 for a case in which the raw material gas having each CO/CO₂ ratio is used, and wherein the ratio of the suppressed heating value and the ratio of the obtained combustion energy increase are values calculated from the following respective equations: ratio of suppressed heating value=(heating value calculated under reference condition-heating value calculated when each raw material gas is used)/heating value calculated under reference condition; and ratio of obtained combustion energy increase=(combustion energy of produced methanol calculated when each raw material gas is used-combustion energy of produced methanol calculated under reference condition) /combustion energy of produced methanol calculated under reference condition, where the reference condition is a case in which a raw material gas containing only carbon dioxide and hydrogen at a ratio "CO₂/H₂" of 1/3 is used, and the combustion energy of the produced methanol is a value calculated from an "equation: combustion energy of produced methanol=production amount of methanol×higher heating value of methanol."
[11] In the production method according to the above-mentioned item [10], the product of the ratio of the suppressed heating value and the ratio of the obtained combustion energy increase may be -3 or more.
[12] According to one embodiment of the present invention, there is provided a liquid fuel synthesis system, including: a reactor storing a catalyst configured to advance a conversion reaction from a raw material gas containing at least a carbon oxide and hydrogen to a liquid fuel; and a raw material gas-supplying portion configured to supply, as the raw material gas, a gas, which contains the carbon oxide, the hydrogen, and an inert gas, and in which a ratio of a concentration of carbon monoxide to a concentration of carbon dioxide in the carbon oxide is 0.9 or less, to the reactor.
[13] In the liquid fuel synthesis system according to the above-mentioned item [12], a concentration of the inert gas in the raw material gas may be 1 vol% or more and 55 vol% or less.
[14] In the liquid fuel synthesis system according to the above-mentioned item [12] or [13], a ratio of a concentration of the inert gas to a concentration of the carbon dioxide in the raw material gas may be 0.05 or more and 1.5 or less.
[15] The liquid fuel synthesis system according to any one of the above-mentioned items [12] to [14] may further include a gas-capturing portion configured to capture an inert gas and carbon dioxide from air or a biogas. A gas containing the inert gas and the carbon dioxide, which is to be supplied from the gas-capturing portion, may be supplied as a constituent component for the raw material gas to the reactor.
[16] In the liquid fuel synthesis system according to any one of the above-mentioned items [12] to [15], the reactor may include a water vapor separation membrane configured to cause water vapor to permeate therethrough.
[17] In the liquid fuel synthesis system according to any one of the above-mentioned items [12] to [15], the reactor may include a liquid fuel separation membrane configured to cause at least the liquid fuel to permeate therethrough.

### Advantageous Effects of Invention

In the method of producing a liquid fuel according to the embodiment of the present invention, the gas containing the carbon oxide, hydrogen, and the inert gas is used as the raw material gas. Thus, the temperature difference between the gas temperature at the inlet of the reactor and the gas temperature at the outlet thereof can be reduced.

### Brief Description of Drawings

FIG. **1** is a schematic view for describing a method of producing a liquid fuel according to one embodiment of the present invention.
FIG. **2** is a schematic view for describing the configuration of an example of a reactor that may be used in the method of producing a liquid fuel according to the embodiment of the present invention.
FIG. **3** is a schematic view for describing the configuration of an example of the reactor that may be used in the method of producing a liquid fuel according to the embodiment of the present invention.
FIGS. **4** are each a schematic view for describing the configuration of an example of the reactor that may be used in the method of producing a liquid fuel according to the embodiment of the present invention.
FIG. **5** is a schematic view for describing the configuration of an example of the reactor that may be used in the method of producing a liquid fuel according to the embodiment of the present invention.
FIG. **6** is a schematic view for describing a liquid fuel synthesis system to be used in the method of producing a liquid fuel according to one embodiment of the present invention.
FIG. **7(a)** and FIG. **7(b)** are each a graph produced on the basis of the results of thermodynamic equilibrium calculation of liquid fuel production when a N₂ ratio is changed for raw material gases having various CO/CO₂ ratios.

### Description of Embodiments

Embodiments of the present invention are described below with reference to the drawings. However, the present invention is not limited to these embodiments. In addition, for clearer illustration, some widths, thicknesses, shapes, and the like of respective portions may be schematically illustrated in the drawings in comparison to the embodiments. However, the widths, the thicknesses, the shapes, and the like are each merely an example, and do not limit the understanding of the present invention.

### A. Method of producing Liquid Fuel

A method of producing a liquid fuel according to an embodiment of the present invention includes:
causing a raw material gas containing at least a carbon oxide and hydrogen to flow into a reactor storing a catalyst (step (I)); and
producing the liquid fuel from the raw material gas through a conversion reaction in the presence of the catalyst (step (II)).

Typically, the raw material gas further contains an inert gas. The ratio of the concentration of carbon monoxide to the concentration of carbon dioxide in the carbon oxide is, for example, 0.9 or less.

The method of producing a liquid fuel according to the embodiment of the present invention typically further includes causing a product gas containing the above-mentioned liquid fuel to flow out of the above-mentioned reactor (step (III)).

In this description, the liquid fuel is a fuel that is in a liquid state at normal temperature and normal pressure or a fuel that can be liquefied under a normal-temperature pressurized state. Examples of the fuel that is in a liquid state at normal temperature and normal pressure include methanol, ethanol, a liquid fuel represented by CₙH₂₍ₘ₋₂ₙ₎ ("m" represents an integer of less than 90 and "n" represents an integer of less than 30), and a mixture thereof. Examples of the fuel that can be liquefied under a normal-temperature pressurized state include propane, butane, and a mixture thereof.

As an example of the above-mentioned conversion reaction, reactions that may occur at the time of the synthesis of methanol through the catalytic hydrogenation of a raw material gas containing carbon monoxide, carbon dioxide, and hydrogen in the presence of a catalyst are represented by the following reaction formulae. In the reaction represented by the formula (2), methanol serving as a product and water serving as a by-product are produced. With regard to a heating value, a heating value when the reaction proceeds to the right-hand side in each formula is described, and a negative sign represents heat generation and a positive sign represents heat absorption.

CO+2H₂ ↔ CH₃OH: -90.8 kJ/mol (1)

CO₂+3H₂ ↔ CH₃OH+H₂O: -49.2 kJ/mol (2)

CO+H₂O ↔ CO₂+H₂: -41.2 kJ/mol (3)

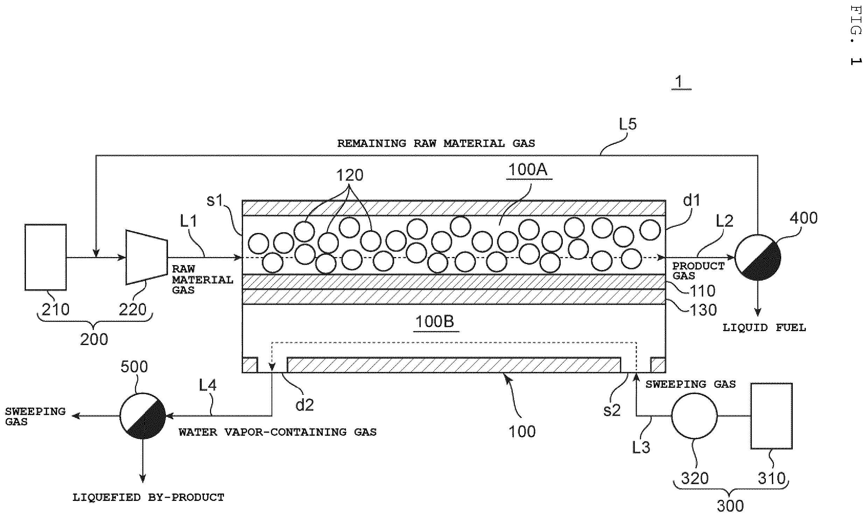

FIG. **1** is a schematic view for describing the method of producing a liquid fuel according to one embodiment of the present invention. The method of producing a liquid fuel according to the embodiment illustrated in FIG. **1** is performed by using a liquid fuel synthesis system **1** including: a reactor **100** storing a catalyst; and a raw material gas-supplying portion **200** configured to supply a raw material gas to the reactor **100.** The liquid fuel synthesis system **1** further includes a sweeping gas-supplying portion **300** that supplies a sweeping gas to the reactor **100.** The raw material gas-supplying portion **200** includes a first gas-storing portion **210** and a pressure-boosting portion **220.** The sweeping gas-supplying portion **300** includes a second gas-storing portion **310** and a heating portion **320.** The method of producing a liquid fuel according to the embodiment of the present invention is described below with reference to FIG. **1****.**

### A-1. Step (I)

In the step (I), the raw material gas containing the carbon oxide, hydrogen, and the inert gas is caused to flow into the reactor storing the catalyst.

The reactor typically includes: a gas inflow port through which the raw material gas is caused to flow into the reactor; a gas outflow port through which the product gas containing the product is caused to flow out of the reactor; and a space that communicates the ports to each other. The catalyst is stored in the space.

The shape of the reactor is not particularly limited, and may be set to, for example, a monolith shape, a flat-plate shape, a tubular shape, a cylindrical shape, a columnar shape, or a polygonal columnar shape. The monolith shape means a shape having a plurality of cells penetrating the shape in its lengthwise direction, and is a concept including a honeycomb shape.

In the embodiment illustrated in FIG. **1****,** the reactor **100** is a so-called membrane reactor, and includes a water vapor separation membrane **110,** a catalyst **120,** a non-permeation-side space **100A,** and a permeation-side space **100B.** In the illustrated example, the water vapor separation membrane **110** is supported by a porous support **130.** The reactor **100** further includes: a first inflow port **s1** and a first outflow port **d1** communicating to each other through the non-permeation-side space **100A;** and a second inflow port **s2** and a second outflow port **d2** communicating to each other through the permeation-side space **100B.** In the reactor **100,** the non-permeation-side space **100A** is a space that stores the catalyst. The reactor **100** preferably has heat resistance and pressure resistance suitable for conditions for the synthesis of a desired liquid fuel.

The water vapor separation membrane **110** causes water vapor, which is a by-product of the conversion reaction from the raw material gas to the liquid fuel, to permeate therethrough. Thus, the reaction equilibrium of the formula (2) can be shifted to a product side through utilization of an equilibrium shift effect.

The molecular diameter (0.26 nm) of water is close to the molecular diameter (0.296 nm) of hydrogen. Accordingly, not only the water vapor that is a by-product of the conversion reaction but also part of hydrogen in the raw material gas can permeate through the water vapor separation membrane 110. In addition, as described later, when helium is used as the inert gas, part of helium in the raw material gas can also permeate through the water vapor separation membrane **110.**

The water vapor separation membrane **110** preferably has a water vapor permeance of 100 nmol/(s·Pa·m²) or more. The water vapor permeance may be determined by an existing method (see Ind. Eng. Chem. Res., 40, 163-175 (2001)).

The water vapor separation membrane **110** preferably has a separation factor of 100 or more. As the separation factor becomes larger, the membrane more easily causes the water vapor to permeate therethrough, and more hardly causes components except the water vapor (e.g., hydrogen, the carbon oxide, the inert gas, and the liquid fuel) to permeate therethrough. The separation factor may be determined by an existing method (see Fig. 1 of "Separation and Purification Technology 239 (2020) 116533").

An inorganic membrane may be used as the water vapor separation membrane **110.** The inorganic membrane is preferred because the membrane has heat resistance, pressure resistance, and water vapor resistance. Examples of the inorganic membrane include a zeolite membrane, a silica membrane, an alumina membrane, and a composite membrane thereof. For example, a LTA zeolite membrane in which the molar ratio (Si/Al) of a silicon element (Si) to an aluminum element (Al) is 1.0 or more and 3.0 or less is suitable because the membrane is excellent in water vapor permeability.

The zeolite membrane to be used as the water vapor separation membrane **110** may be obtained by a production method described in, for example, JP 2004-66188 A. In addition, the silica membrane to be used as the water vapor separation membrane **110** may be obtained by a production method described in, for example, WO 2008/050812 A1.

The porous support **130** includes a porous material. For example, a ceramic material, a metal material, a resin material, and a composite member thereof may each be used as the porous material, and the ceramic material is particularly suitable. For example, alumina (Al₂O₃) , titania (TiO₂), mullite (Al₂O₃·SiO₂) , ceramic fragments, cordierite (Mg₂Al₄Si₅O₁₈) , and a composite material containing two or more of these materials may each be used as an aggregate for the ceramic material, and alumina is suitable in consideration of ease of availability, green body stability, and corrosion resistance. At least one of titania, mullite, easily sinterable alumina, silica, glass frit, a clay mineral, or easily sinterable cordierite may be used as an inorganic binder for the ceramic material. The ceramic material may be free of any inorganic binder.

The average pore diameter of the porous support may be set to 5 µm or more and 25 µm or less. The average pore diameter of the porous support may be measured by a mercury intrusion method. The porosity of the porous support may be set to 25% or more and 50% or less. The average particle diameter of the porous material for forming the porous support may be set to 1 µm or more and 100 µm or less. In this embodiment, the average particle diameter is the arithmetic average value of the maximum diameters of 30 measurement object particles (randomly selected) measured by sectional microstructure observation with a scanning electron microscope (SEM).

The catalyst **120** advances the conversion reaction from the raw material gas to the liquid fuel. The catalyst is arranged in the non-permeation-side space **100A.** Although the catalyst is preferably filled into the non-permeation-side space **100A,** the catalyst may be arranged in a layer shape or an island shape on the surface of the water vapor separation membrane **110.** When the catalyst has a particle shape like the illustrated example, the particle diameters (diameters) of catalyst particles may each be set to, for example, 0.5 mm or more and 10 mm or less. Each of the catalyst particles may be formed only of the catalyst, or may have a configuration in which the catalyst is carried by a carrier particle. The carrier particle is preferably a porous particle.

Any catalyst suitable for a conversion reaction to a desired liquid fuel may be used as the catalyst. Specifically, metal catalysts (e.g., copper and palladium), oxide catalysts (e.g., zinc oxide, zirconia, and gallium oxide), and catalysts obtained by compositing these catalysts (e.g., copper-zinc oxide, copper-zinc oxide-alumina, copper-zinc oxide-chromium oxide-alumina, copper-cobalt-titania, and catalysts obtained by modifying these catalysts with palladium) may each be used.

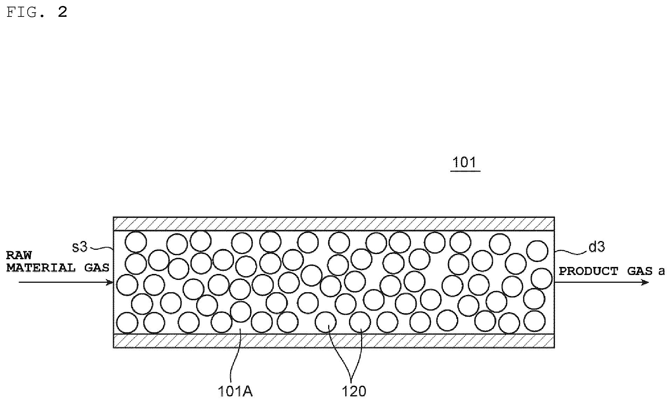

The reactor is not limited to the above-mentioned illustrated example. For example, as illustrated in FIG. **2****,** a reactor **101** may be a flow reactor including: a gas inflow port **s3;** a gas outflow port **d3;** a space **101A** that communicates the ports to each other; and the catalyst (catalyst particles) **120** arranged (filled) in the space **101A.** The raw material gas containing the carbon oxide, hydrogen, and the inert gas is caused to flow into the reactor **101** through the gas inflow port **s3,** and a product gas (product gas **"a"**) containing the liquid fuel, a by-product, and an unreacted raw material gas (remaining raw material gas) is caused to flow out of the reactor through the gas outflow port **d3.**

As described above, the raw material gas contains the carbon oxide, hydrogen, and the inert gas. At least carbon dioxide is used as the carbon oxide, and carbon monoxide may be further used to the extent that the effect of the present invention is obtained. The raw material gas preferably contains carbon dioxide at a ratio higher than that of carbon monoxide. When the ratio (CO vol%/CO₂ vol%, hereinafter sometimes referred to as "CO/CO₂ ratio") of the concentration of carbon monoxide to the concentration of carbon dioxide in the carbon oxide in the raw material gas is large, a suppressing effect on temperature unevenness in the reactor in the case where the inert gas is blended is weak, and hence the effect of the present invention is not sufficiently obtained in some cases. Specifically, when the above-mentioned CO/CO₂ ratio is large, the suppressing effect on the temperature unevenness in the reactor in the case where the inert gas is blended weakens because of the following reasons: when the reaction represented by the formula (1) and the reaction represented by the formula (2) are compared to each other, the reaction represented by the formula (1) has the larger heating value; and the equilibrium conversion ratio of the reaction represented by the formula (1) is larger than that of the reaction represented by the formula (2), and hence the heating value further increases. The above-mentioned CO/CO₂ ratio is typically 0.9 or less. In this case, as the inlet temperature of the reactor reduces, the equilibrium conversion ratio increases, and hence a temperature change due to heat generation becomes larger. Accordingly, from the viewpoints of the equilibrium conversion ratio and the temperature unevenness, when the reactor is operated at an inlet temperature of 250°C or less, the CO/CO₂ ratio is preferably 0.5 or less, and when the reactor is operated at an inlet temperature of 230°C or less, the CO/CO₂ ratio is preferably 0.2 or less. The lower limit value of the CO/CO₂ ratio is not particularly limited because after the passage of the catalyst, the reaction represented by the formula (3) occurs. However, the CO/CO₂ ratio is preferably set to 0.02 or more from the viewpoint of the ease with which the ratio is controlled. The above-mentioned CO/CO₂ ratio is a CO/CO₂ ratio in the raw material gas at the inlet (specifically, the first inflow port **s1**) of the reactor, and when the remaining raw material gas is recycled, the ratio is a CO/CO₂ ratio in a raw material gas prepared by using the remaining raw material gas. In addition, other descriptions concerning, for example, the contents and ratios of the respective gases to be incorporated into the raw material gas are descriptions about the raw material gas at the inlet of the reactor.

The concentration of the carbon oxide in the raw material gas (when the gas contains carbon monoxide and carbon dioxide, their total concentration) is, for example, 5 vol% or more and 50 vol% or less, preferably 10 vol% or more and 40 vol% or less. In one embodiment, the concentration of carbon dioxide in the raw material gas is, for example, 5 vol% or more and 50 vol% or less, preferably 9 vol% or more and 35 vol% or less. In one embodiment, the concentration of carbon monoxide in the raw material gas is, for example, 23 vol% or less, preferably 20 vol% or less. The concentration of hydrogen in the raw material gas is, for example, 20 vol% or more and 85 vol% or less, preferably 30 vol% or more and 80 vol% or less.

Examples of the inert gas include nitrogen, helium, and argon. Those gases may be used alone or in combination thereof. The concentration of the inert gas in the raw material gas is, for example, 1 vol% or more, preferably 2 vol% or more, more preferably 3 vol% or more, still more preferably 5 vol% or more, and is, for example, 55 vol% or less, preferably 30 vol% or less, more preferably 10 vol% or less. When the concentration of the inert gas is less than 1 vol%, the temperature unevenness in the reactor cannot be sufficiently suppressed, and hence the effect of the present invention is not sufficiently obtained in some cases. When the concentration of the inert gas is more than 55 vol%, the concentration of a reaction component reduces, and hence a target liquid fuel is not sufficiently obtained in some cases. In one embodiment, the ratio (inert gas vol%/CO₂ vol%, hereinafter sometimes referred to as "Inert/CO₂ ratio") of the concentration of the inert gas to the concentration of carbon dioxide in the raw material gas is, for example, 0.05 or more, preferably 0.15 or more. From the viewpoint of controlling a detector or a mixing valve, the Inert/CO₂ ratio is preferably set to 0.15 or more because it becomes easier to control the Inert/CO₂ ratio. The Inert/CO₂ ratio in the raw material gas is, for example, 1.5 or less, preferably 1.1 or less. When the Inert/CO₂ ratio is small, the temperature unevenness in the reactor cannot be sufficiently suppressed, and hence the effect of the present invention is not sufficiently obtained in some cases. When the Inert/CO₂ ratio is large, the concentration of the reaction component reduces, and hence the production amount of the liquid fuel becomes insufficient in some cases. In addition, a case in which the Inert/CO₂ ratio is 1.1 or less may be advantageous from the viewpoint of reducing raw material loss because the ratio at which the remaining raw material gas is discharged to the outside of the system by purging at the time of its recycling can be suitably suppressed.

As in the Inert/CO₂ ratio, from the viewpoint of achieving both the suppressing effect on the temperature unevenness in the reactor and the production amount of the liquid fuel, the ratio ((inert gas vol%+CO vol%)/CO₂ vol%) of the total of the concentration of the inert gas and the concentration of carbon monoxide to the concentration of carbon dioxide in the raw material gas is, for example, 0.05 or more, and is, for example, 2.4 or less. The above-mentioned ratio is preferably 0.07 or more from the viewpoint of the ease with which the ratio is controlled, and the ratio is preferably 2.2 or less from the viewpoint of reducing the raw material loss. In addition, from the viewpoints of the equilibrium conversion ratio and the temperature unevenness, when the reactor is operated at an inlet temperature of, for example, 250°C or less, the above-mentioned ratio is preferably 2.2 or less.

The total concentration of the carbon oxide, hydrogen, and the inert gas in the raw material gas is, for example, 80 vol% or more and 100 vol% or less, preferably 96 vol% or more and 100 vol% or less. The raw material gas may be formed substantially only of the carbon oxide, hydrogen, and the inert gas.

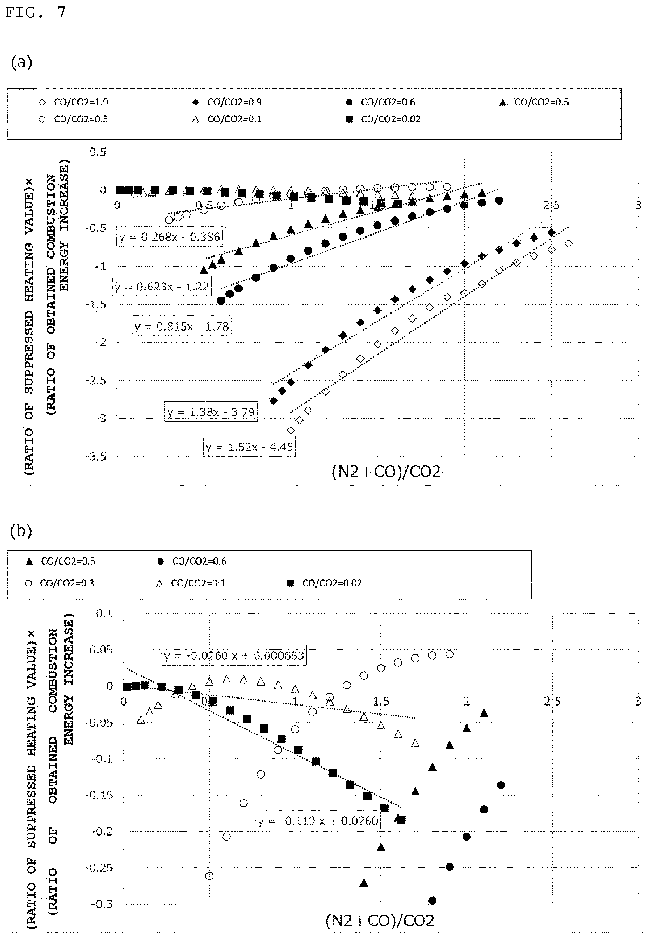

FIG. **7(a)** is a graph produced with Excel on the basis of the results of thermodynamic equilibrium calculation performed on the reactions represented by the formulae (1) to (3) when a N₂ ratio is changed for raw material gases having various CO/CO₂ ratios, and FIG. **7(b)** is a graph obtained by enlarging the range of Y=-0.3 to 0.1 in the graph of FIG. **7(a)****.** In each of the graphs, an X-axis indicates the ratio " (N₂+CO) /CO₂" in each of the raw material gases, and a Y-axis indicates the product "(ratio of suppressed heating value)×(ratio of obtained combustion energy increase)."

In the production of each of the above-mentioned graphs, a case in which a raw material gas containing only CO₂ and H₂ at a ratio "CO₂/H₂" of 1/3 (CO₂ vol%+H₂ vol%=100 vol%, CO/CO₂ ratio=0, (N₂+CO)/CO₂ ratio=0) is used is defined as a reference condition, and the ratio "(heating value calculated under reference condition-heating value calculated when each raw material gas is used)/heating value calculated under reference condition" is defined as the "ratio of suppressed heating value," and the ratio "(combustion energy of produced methanol calculated when each raw material gas is used-combustion energy of produced methanol calculated under reference condition) /combustion energy of produced methanol calculated under reference condition" is defined as the "ratio of obtained combustion energy increase." Herein, the "combustion energy of produced methanol" is calculated from the product "production amount of methanol×higher heating value (HHV) of methanol."

In each of the graphs, a total of 18 pieces of data (i.e., data when a N₂/CO₂ ratio is changed in the range of from 0 to 1.6 in increments of 0.1, and when the N₂/CO₂ ratio=0.05) are plotted for a case in which the raw material gas having each CO/CO₂ ratio is used.

In each of the graphs, the X-axis is an indicator factoring in both the ratio of N₂ related to the suppression of a heating value and the ratio of CO related to the production amount of the liquid fuel. In a graph produced by the above-mentioned production method on the basis of the results of thermodynamic equilibrium calculation performed under the following conditions, the value "a" of a gradient when an approximate straight line is produced by subjecting 18 points plotted for the raw material gas having each CO/CO₂ ratio to linear approximation (Y=aX+b) is preferably 1.4 or less. Although the lower limit of the value "a" is not particularly limited, the lower limit may be, for example, -0.2 or more. When the value "a" is more than 1.4, it may become difficult to simultaneously achieve both the suppressing effect on the temperature unevenness in the reactor and the production amount of the liquid fuel. In addition, the value "a" is preferably 0.7 or less from the viewpoint of making it easier to achieve both the suppressing effect on the temperature unevenness in the reactor and the production amount of the liquid fuel.

The temperature unevenness in the reactor may result from heat generated by a synthesis reaction for the liquid fuel. Accordingly, the suppression of the synthesis reaction for the liquid fuel through the dilution of the raw material gas with the inert gas (consequently, the suppression of the heating value of the reaction) can contribute to the suppression of the temperature unevenness in the reactor. Meanwhile, when the synthesis reaction for the liquid fuel is suppressed, the production amount of the liquid fuel may reduce. Accordingly, the Y-axis of each of the graphs may be used as an indicator of a balance between the suppressing effect on the temperature unevenness and the production amount of the liquid fuel. Specifically, the suppressing effect on the temperature unevenness and the production amount of the liquid fuel are in a trade-off relationship. When one of the effect or the amount has a positively large value, the other thereof has a negatively large value, and hence the product of the effect and the amount tends to deviate from 0. Accordingly, as the value of the Y-axis becomes closer to 0, it can be judged that the balance between the effect and the amount is kept to a larger extent. In the graph produced by the above-mentioned production method on the basis of the results of the thermodynamic equilibrium calculation performed under the following conditions, the value of the Y-axis is preferably -3 or more, more preferably -1.5 or more, and although its upper limit is not particularly limited, the upper limit may be, for example, 0.1 or less. When the value of the Y-axis is less than -3, the simultaneous achievement of both the suppressing effect on the temperature unevenness in the reactor and the production amount of the liquid fuel may become insufficient.

As described above, when liquid fuel production is performed by using a raw material gas having such composition that the value "a" of the gradient of the approximate straight line in the above-mentioned graph becomes preferably 1.4 or less, more preferably 0.7 or less, or by using preferably a raw material gas having such composition that the value "a" of the gradient becomes 1.4 or less, or 0.7 or less, and the value of the Y-axis becomes -3 or more, the balance between the suppressing effect on the temperature unevenness and the production amount of the liquid fuel can be satisfactorily kept. For example, according to the above-mentioned graph, when the CO/CO₂ ratio in the raw material gas is 0.9 or less, the value of the Y-axis falls within the range of -3 or more, and hence the liquid fuel can be produced while the balance between the suppressing effect on the temperature unevenness and the production amount of the liquid fuel is kept. In addition, it is found that the blending of the raw material gas with N₂ can improve the balance. Further, it is also found that when the CO/CO₂ ratio is 0.9 or less, the value "a" of the gradient of the approximate straight line is 1.4 or less, and hence the balance between the suppressing effect on the temperature unevenness and the production amount of the liquid fuel can be satisfactorily kept. Reaction conditions for the method of producing a liquid fuel according to the embodiment of the present invention are not limited to the following reaction conditions used in the thermodynamic equilibrium calculation.

The above-mentioned thermodynamic equilibrium calculation was performed under the following conditions with the database of thermodynamic data built in chemical equilibrium calculation software "MALT". However, the thermodynamic data to be used is not limited thereto, and for example, the database of the NIST Chemistry web book made public by the National Institute of Standards and Technology (NIST) may be used.

| | |
|---|---|
| ·Reaction temperature: | 200°C |
| ·Reaction pressure: | 4 MPa (G) |
| ·Raw material gas composition: | H₂/CO₂=3 |
| | : N₂/CO₂=in the range of from 0 to 1.6 in increments of 0.1, and 0.05 |
| | : CO/CO₂=0.02, 0.1, 0.3, 0.5, 0.6, 0.9, or 1.0 |

The raw material gas is prepared by mixing the respective components so that desired composition may be obtained. In one embodiment, the raw material gas is prepared by using a carbon dioxide-enriched gas captured from air by direct air capture (DAC). The carbon dioxide-enriched gas obtained by the DAC contains an inert gas (typically, nitrogen) and carbon dioxide captured from the air. Specifically, the carbon dioxide-enriched gas obtained by the DAC may contain a small amount (e.g., 0.05 vol% or more and 60 vol% or less) of nitrogen derived from the air in addition to a high concentration (e.g., 30 vol% or more and 99.9 vol% or less) of carbon dioxide. In addition, the concentration of carbon monoxide in the carbon dioxide-enriched gas is low. Accordingly, when the raw material gas is prepared by using the carbon dioxide-enriched gas obtained by the DAC, the following advantage is obtained: there is no need to separately prepare and mix an inert gas; or the amount of the inert gas to be separately prepared can be reduced. In another embodiment, the raw material gas is prepared by using a carbon dioxide-enriched gas captured from a biogas. The biogas is a gas produced through fermentation (methane fermentation) by using biomass, such as garbage, waste paper, or livestock manure, as a raw material. The main components of the biogas are methane and carbon dioxide, and the biogas may further contain nitrogen or the like. The use of a separation membrane can capture carbon dioxide from the biogas to provide the carbon dioxide-enriched gas. The carbon dioxide-enriched gas captured from the biogas may contain an inert gas (typically, nitrogen) in addition to carbon dioxide, and the concentration of carbon monoxide therein is low. Accordingly, when the raw material gas is prepared by using the carbon dioxide-enriched gas captured from the biogas, the following advantage is obtained: there is no need to separately prepare and mix an inert gas; or the amount of the inert gas to be separately prepared can be reduced. In one embodiment, the liquid fuel synthesis system 1 may be configured as follows: the system further includes a gas-capturing portion configured to capture an inert gas (typically, nitrogen) and carbon dioxide from the air through the DAC, or a gas-capturing portion configured to capture an inert gas (typically, nitrogen) and carbon dioxide from the biogas with the separation membrane, and the carbon dioxide-enriched gas (gas containing the inert gas and carbon dioxide captured from the air or the biogas) is supplied from the gas-capturing portion to the raw material gas-supplying portion. The carbon dioxide-enriched gas may be directly supplied from the gas-capturing portion to the raw material gas-supplying portion, or may be supplied to the raw material gas-supplying portion after having passed as a constituent component for a sweeping gas through the reactor. For example, the gas-capturing portion includes a carbon dioxide adsorbent (e.g., an amine compound), which adsorbs carbon dioxide by being brought into contact with a carbon dioxide-containing gas, and which desorbs carbon dioxide through heating, a pressure reduction, or the like, and the portion can capture a gas containing the inert gas and a high concentration of carbon dioxide from the air with the carbon dioxide adsorbent. In addition, for example, the gas-capturing portion includes a separation membrane such as a carbon dioxide-permeable membrane, and can capture a carbon dioxide-enriched gas containing the inert gas and a high concentration of carbon dioxide from the carbon dioxide-containing gas such as a biogas.

The raw material gas is flowed into the reactor at a desired temperature and a desired pressure. In the embodiment illustrated in FIG. 1, the gas containing the carbon oxide, hydrogen, and the inert gas, the gas being stored in the first gas-storing portion 210, is mixed with the remaining raw material gas, and the pressure and temperature of the mixture are increased in the pressure-boosting portion **220.** After that, the mixture is flowed as the raw material gas into the non-permeation-side space **100A** of the reactor **100** through a line **L1.** The pressure-boosting portion **220** includes, for example, a compressor or a pressure booster. In the illustrated example, the raw material gas prepared in desired composition in advance is supplied from the first gas-storing portion **210** to the reactor. However, the raw material gas only needs to be flowed in the desired composition into the reactor, and hence the time point at which the desired composition is achieved is not limited to the embodiment of the illustrated example. For example, the carbon oxide, hydrogen, and the inert gas may be separately caused to flow from their respective supply sources into the reactor.

The temperature of the raw material gas when the gas is caused to flow into the reactor (in other words, the temperature of the raw material gas at the gas inflow port of the reactor) is, for example, 40°C or more and 350°C or less, preferably 180°C or more and 330°C or less.

The pressure of the raw material gas when the gas is caused to flow into the reactor (in other words, the pressure thereof at the gas inflow port of the reactor) is, for example, 1.0 MPa (G) or more and 6.0 MPa (G) or less, preferably 2.0 MPa (G) or more and 6.0 MPa (G) or less.

The flow rate of the raw material gas to be caused to flow into the reactor is, for example, 1,000/h or more and 50,000/h or less, preferably 2,000/h or more and 20,000/h or less, more preferably 3,000/h or more and 12,000/h or less in terms of space velocity GHSV.

### A-2. Step (II)

In the step (II), in the presence of the catalyst, the liquid fuel is produced from the raw material gas by the conversion reaction. As represented by, for example, the reaction formulae (1) and (2), the carbon oxide and hydrogen are subjected to catalytic hydrogenation in the presence of the catalyst to produce methanol. Each of the produced liquid fuel and a by-product (typically, water) is in a gas state at the time of its synthesis, and at least until the liquid fuel or the by-product flows out of the reactor, the liquid fuel or the by-product is maintained while being in a gas state.

According to the embodiment illustrated in FIG. **1****,** the raw material gas passes through the first inflow port **s1** to flow into the non-permeation-side space **100A,** and the catalyst **120** advances the conversion reaction from the raw material gas to the liquid fuel. Thus, the liquid fuel is synthesized. In addition, water vapor that is a by-product and hydrogen (and an inert gas depending on the kind) in the raw material gas permeate through the water vapor separation membrane **110** to flow into the permeation-side space **100B.** The sweeping gas for sweeping the water vapor and hydrogen is flowed from the second inflow port **s2** into the permeation-side space **100B** through a line **L3.** Specifically, the sweeping gas stored in the second gas-storing portion **310** is heated to a desired temperature (e.g., 150°C or more and 350°C or less) in the heating portion **320,** and is then flowed into the permeation-side space **100B** of the reactor **100** through the line **L3.** Any appropriate gases, such as nitrogen, a carbon oxide, hydrogen, and a mixed gas thereof, may each be used as the sweeping gas.

Each of the reactions represented by the reaction formulae (1) to (3) is an equilibrium reaction, and hence, to increase both of its conversion ratio and reaction rate, the reaction is preferably performed under high temperature and high pressure. The reaction temperature (in other words, the temperature of the space of the reactor having stored therein the catalyst) is, for example, 180°C or more, preferably 200°C or more and 350°C or less, more preferably 200°C or more and 300°C or less. The reaction pressure (in other words, the pressure of the space of the reactor having stored therein the catalyst) is, for example, 1 MPa (G) or more, preferably 2.0 MPa (G) or more and 6.0 MPa (G) or less, more preferably 2.5 MPa (G) or more and 4.0 MPa (G) or less.

In each of the reactions represented by the formulae (1) and (2), the production of methanol is an exothermic reaction, and hence a temperature difference (temperature unevenness) may occur in the reactor owing to generated heat or a gas flow. When such temperature difference occurs, the equilibrium conversion ratio of the reaction reduces in a high-temperature portion, and hence a reaction yield reduces in some cases. In contrast, according to the production method of the embodiment of the present invention, an excessive increase in temperature in the reactor is suppressed by diluting the raw material gas with the inert gas, and hence a temperature difference between a gas temperature at the inlet of the reactor and a gas temperature at the outlet thereof can be reduced. Thus, the uniformity of the temperature of the reactor can be improved, and hence a reduction in conversion ratio can be suppressed.

### A-3. Step (III)

In the step (III), the product gas containing the liquid fuel is caused to flow out of the reactor. The product gas typically contains the liquid fuel and the remaining raw material gas. Accordingly, the liquid fuel is preferably separated and recovered from the product gas.

The temperature of the product gas when the gas is caused to flow out of the reactor (in other words, the temperature of the product gas at the gas outflow port of the reactor) is, for example, 200°C or more and 350°C or less, preferably 200°C or more and 300°C or less.

A difference (gas temperature at gas outflow port-gas temperature at gas inflow port) between a gas temperature at the gas inflow port and a gas temperature at the gas outflow port is, for example, 10°C or more and 100°C or less, preferably 20°C or more and 50°C or less. Herein, the gas temperature at the gas inflow port is a gas temperature at a position distant inward from the gas inflow port by 20% of the total length of the reactor, and the gas temperature at the gas outflow port is a gas temperature at a position directly behind the gas outflow port.

In the embodiment illustrated in FIG. **1****,** the product gas passes through the first outflow port **d1** to be flowed out of the reactor **100** (more specifically, the non-permeation-side space **100A)** into a line **L2.** In addition, the water vapor, hydrogen, and the like that have permeated through the water vapor separation membrane **110** to flow into the permeation-side space **100B** pass through the second outflow port **d2** together with the sweeping gas to be flowed as a water vapor-containing gas out of the reactor **100** (more specifically, the permeation-side space **100B**) into a line **L4.**

The product gas that has been flowed out of the reactor **100** is flowed into a drain trap **400** arranged downstream of the reactor **100.** The drain trap **400** liquefies the liquid fuel in a gas state, and separates the remaining raw material gas from the liquid fuel. Thus, the liquid fuel is recovered. Meanwhile, the remaining raw material gas thus separated is returned to the upstream of the reactor by a line **L5,** and is mixed with the gas that has been flowed out of the first gas-storing portion. Thus, the raw material gas is obtained. From the viewpoint of adjusting the composition of the raw material gas, the remaining raw material gas may be purged as required. The recycling rate of the remaining raw material gas in the raw material gas (volume percentage of the remaining raw material gas in the raw material gas) may be, for example, 15% or more and 85% or less. The resultant raw material gas is subjected to a pressure increase and a temperature increase in the pressure-boosting portion **220,** and is flowed into the reactor **100** through the line **L1.** As described above, in the embodiment of the present invention, the concentration ratios of the respective gases in the raw material gas are suitably controlled, and hence even when the remaining raw material gas is recycled, the liquid fuel can be continuously produced while both the suppressing effect on the temperature unevenness in the reactor and the production amount of the liquid fuel are achieved.

With regard to the water vapor-containing gas that has been caused to flow out of the reactor **100,** similarly, a condensable by-product (typically, water vapor) is liquefied, and is separated and captured from the remaining gas by a drain trap **500.** The following may be performed as required: hydrogen or the like is recovered from the separated gas, and is reused as part of the raw material gas.

### B. Modification Examples

The present invention is not limited to the above-mentioned embodiments, and various modifications may be made thereto. For example, the configurations described in the above-mentioned embodiments may each be replaced by substantially the same configuration, a configuration having the same action and effect, and a configuration that can achieve the same object.

### B-1. Modification Example 1

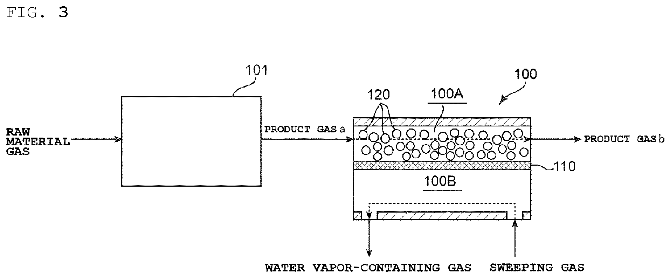

The reactor may be obtained by combining a plurality of reactors. For example, as illustrated in FIG. **3****,** the reactor may have a configuration in which the flow reactor **101** illustrated in FIG. **2** is arranged upstream of the reactor **100** that is a membrane reactor. The reactor **100** includes the water vapor separation membrane **110,** the catalyst **120,** the non-permeation-side space **100A,** and the permeation-side space **100B.** The water vapor separation membrane **110** is typically supported by a porous support (not shown). According to the reactor having such configuration, the product gas **"a"** that has flowed out of the reactor **101,** the gas containing the liquid fuel, the water vapor, and the remaining raw material gas, flows into the non-permeation-side space **100A** of the reactor **100,** and hence a conversion reaction from the remaining raw material gas to the liquid fuel proceeds. A product gas **"b"** containing the liquid fuel produced in the reactors **101** and **100** flows out of the non-permeation-side space **100A.** The water vapor that has permeated through the water vapor separation membrane **110** and the sweeping gas flow as a water vapor-containing gas out of the permeation-side space **100B.**

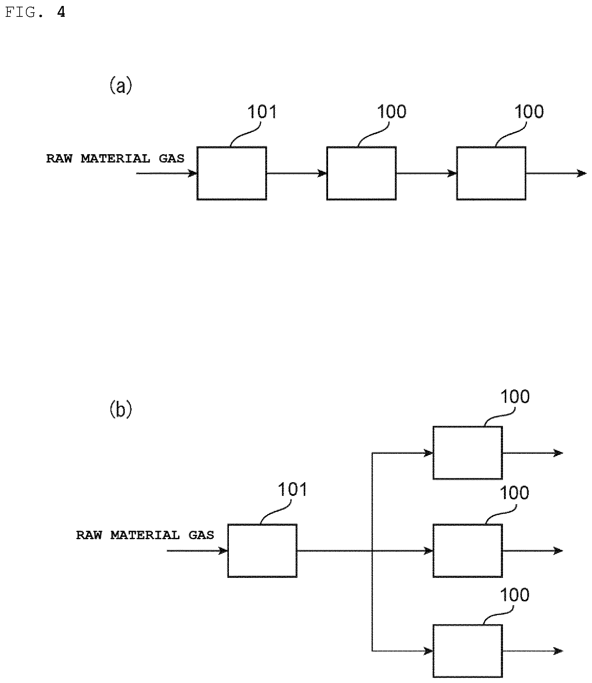

The number of the reactors **100** to be arranged downstream of the flow reactor **101** is not limited, and as illustrated in each of FIGS. **4****,** the two or more reactors 100 may be connected in series and/or in parallel. In each of the figures, with regard to the downstream of the reactor 101, only the flow of the product gas is illustrated.

### B-2. Modification Example 2

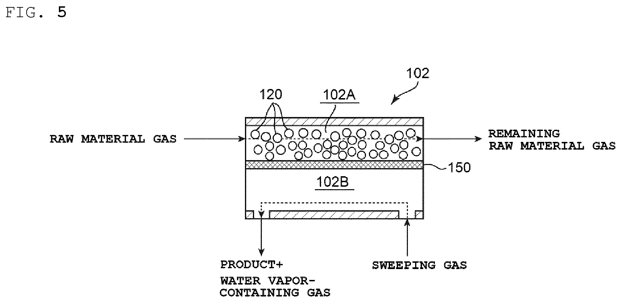

The reactor of the above-mentioned embodiment has the following configuration: the water vapor separation membrane that causes water vapor to permeate therethrough is used as a separation membrane, which separates the water vapor and the liquid fuel from each other, to cause the water vapor to permeate from the non-permeation-side space to the permeation-side space, thereby separating the water vapor. However, the reactor may have the following configuration: a liquid fuel separation membrane that causes the liquid fuel to permeate therethrough is used to cause the liquid fuel to permeate from the non-permeation-side space to the permeation-side space, thereby separating the liquid fuel. For example, a reactor **102** illustrated in FIG. **5** includes a liquid fuel separation membrane **150,** the catalyst **120,** a non-permeation-side space **102A,** and a permeation-side space **102B.** The liquid fuel separation membrane 150 may be typically supported by a porous support (not shown). According to such configuration, the liquid fuel permeates from the non-permeation-side space **102A** to the permeation-side space **102B,** and hence the reaction equilibrium of each of the formulae (1) and (2) can be shifted to a product side. A membrane described in JP 2020-23488 A may be used as a separation membrane that selectively causes the liquid fuel to permeate therethrough. In the present invention, the separation membrane that separates the water vapor and the liquid fuel from each other has higher selective permeability for one of the water vapor and the liquid fuel than for the other thereof, and may not completely separate both the water vapor and the liquid fuel from each other as long as the effect of the present invention is obtained. For example, the liquid fuel separation membrane causes the liquid fuel to permeate therethrough with selectivity higher than that of the water vapor, and does not completely separate both the water vapor and the liquid fuel from each other.

### B-3. Modification Example 3

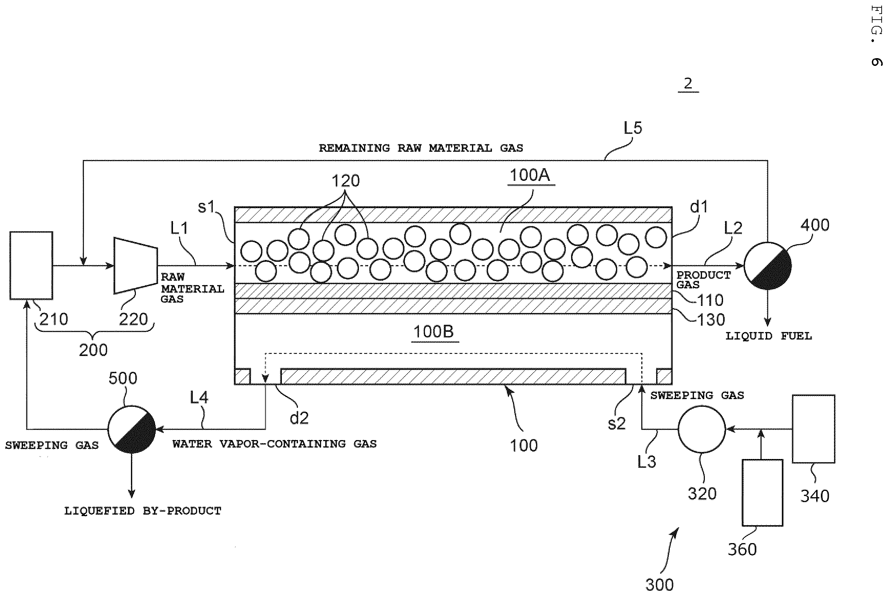

As described above, the raw material gas may be prepared by using a carbon dioxide-enriched gas captured from air by the DAC or from a biogas with a separation membrane (e.g., a gas containing the inert gas and carbon dioxide captured from the air or from the biogas). Accordingly, the liquid fuel synthesis system to be used in the method of producing a liquid fuel according to the embodiment of the present invention may be configured as follows: the system further includes a gas-capturing portion configured to capture an inert gas and carbon dioxide from the air by the DAC, or from the biogas with the separation membrane, and the carbon dioxide-enriched gas to be supplied from the gas-capturing portion is supplied as a constituent component for the raw material gas to the reactor. For example, like a liquid fuel synthesis system **2** illustrated in FIG. **6****,** the following configuration is permitted: the sweeping gas-supplying portion **300** includes a gas-capturing portion **340** and a hydrogen-producing portion **360,** and a sweeping gas prepared by mixing the carbon dioxide-enriched gas supplied from the gas-capturing portion **340** and hydrogen supplied from the hydrogen-producing portion **360** is supplied as a constituent component for the raw material gas to the reactor **100.** In Modification Example 3, the total amount of carbon dioxide to be incorporated into the raw material gas may be derived from the carbon dioxide-enriched gas captured in the gas-capturing portion **340,** or only part thereof may be derived from the carbon dioxide-enriched gas.

The gas-capturing portion **340** typically includes a carbon dioxide adsorbent (e.g., an amine compound), which adsorbs carbon dioxide by being brought into contact with a carbon dioxide-containing gas, and which desorbs carbon dioxide through heating, a pressure reduction, or the like, and the portion can capture a carbon dioxide-enriched gas containing the inert gas and a high concentration of carbon dioxide from the air with the carbon dioxide adsorbent. Alternatively, the gas-capturing portion **340** includes a separation membrane such as a carbon dioxide-permeable membrane, and can capture a carbon dioxide-enriched gas containing the inert gas and a high concentration of carbon dioxide from the carbon dioxide-containing gas such as a biogas. The hydrogen-producing portion **360** is, for example, a hydrogen-producing facility utilizing a water electrolysis technology.

### Industrial Applicability

The method of producing a liquid fuel according to the embodiment of the present invention may be suitably used in the production of a liquid fuel such as methanol.

### Reference Signs List

**100** reactor
**100A** non-permeation-side space
**100B** permeation-side space
**110** water vapor separation membrane
**120** catalyst
**200** raw material gas-supplying portion
**300** sweeping gas-supplying portion

## Claims

1. A method of producing a liquid fuel, comprising:
causing a raw material gas containing at least a carbon oxide and hydrogen to flow into a reactor storing a catalyst; and
producing the liquid fuel from the raw material gas through a conversion reaction in the presence of the catalyst,
wherein the raw material gas further contains an inert gas, and
wherein a ratio of a concentration of carbon monoxide to a concentration of carbon dioxide in the carbon oxide is 0.9 or less.

2. The production method according to claim 1, wherein a concentration of the inert gas in the raw material gas is 1 vol% or more and 55 vol% or less.

3. The production method according to claim 1, wherein a ratio of a concentration of the inert gas to a concentration of the carbon dioxide in the raw material gas is 0.05 or more and 1.5 or less.

4. The production method according to claim 1, wherein a concentration of the carbon oxide in the raw material gas is 5 vol% or more and 50 vol% or less.

5. The production method according to claim 1, wherein a concentration of the hydrogen in the raw material gas is 20 vol% or more and 85 vol% or less.

6. The production method according to claim 1, wherein the raw material gas has a temperature of 40°C or more and 350°C or less when caused to flow into the reactor.

7. The production method according to claim 1, further comprising preparing the raw material gas by using a gas containing an inert gas and carbon dioxide captured from air or a biogas.

8. The production method according to any one of claims 1 to 7, wherein the reactor includes a water vapor separation membrane configured to cause water vapor to permeate therethrough.

9. The production method according to any one of claims 1 to 7, wherein the reactor includes a liquid fuel separation membrane configured to cause at least the liquid fuel to permeate therethrough.

10. A method of producing a liquid fuel, comprising:
causing a raw material gas containing at least a carbon oxide and hydrogen to flow into a reactor storing a catalyst; and
producing the liquid fuel from the raw material gas through a conversion reaction in the presence of the catalyst,
wherein the raw material gas further contains an inert gas,
wherein the liquid fuel contains methanol,
wherein an approximate straight line produced by subjecting points plotted on the basis of results of thermodynamic equilibrium calculation, which is performed on the conversion reaction at 200°C and 4 MPa (G) when a ratio of a concentration of carbon monoxide (CO) to a concentration of carbon dioxide (CO₂) in the carbon oxide in the raw material gas and a ratio of a concentration of the inert gas (Inert) to a concentration of the carbon dioxide (CO₂) in the raw material gas are changed, against an X-axis indicating a ratio "(Inert+CO)/CO₂" in the raw material gas and a Y-axis indicating a product "(ratio of suppressed heating value)×(ratio of obtained combustion energy increase)" to linear approximation (Y=aX+b) has a gradient "a" of 1.4 or less,
wherein the plotting is performed on a total of 18 pieces of data when an Inert/CO₂ ratio is changed in a range of from 0 to 1.6 in increments of 0.1, and when the Inert/CO₂ ratio is 0.05 for a case in which the raw material gas having each CO/CO₂ ratio is used, and
wherein the ratio of the suppressed heating value and the ratio of the obtained combustion energy increase are values calculated from the following respective equations:
ratio of suppressed heating value=(heating value calculated under reference condition-heating value calculated when each raw material gas is used)/heating value calculated under reference condition; and
ratio of obtained combustion energy increase=(combustion energy of produced methanol calculated when each raw material gas is used-combustion energy of produced methanol calculated under reference condition) /combustion energy of produced methanol calculated under reference condition, where
the reference condition is a case in which a raw material gas containing only carbon dioxide and hydrogen at a ratio "CO₂/H₂" of 1/3 is used, and
the combustion energy of the produced methanol is a value calculated from an "equation: combustion energy of produced methanol=production amount of methanol×higher heating value of methanol."

11. The production method according to claim 10, wherein the product of the ratio of the suppressed heating value and the ratio of the obtained combustion energy increase is -3 or more.

12. A liquid fuel synthesis system, comprising:
a reactor storing a catalyst configured to advance a conversion reaction from a raw material gas containing at least a carbon oxide and hydrogen to a liquid fuel; and
a raw material gas-supplying portion configured to supply, as the raw material gas, a gas, which contains the carbon oxide, the hydrogen, and an inert gas, and in which a ratio of a concentration of carbon monoxide to a concentration of carbon dioxide in the carbon oxide is 0.9 or less, to the reactor.

13. The liquid fuel synthesis system according to claim 12, wherein a concentration of the inert gas in the raw material gas is 1 vol% or more and 55 vol% or less.

14. The liquid fuel synthesis system according to claim 12, wherein a ratio of a concentration of the inert gas to a concentration of the carbon dioxide in the raw material gas is 0.05 or more and 1.5 or less.

15. The liquid fuel synthesis system according to claim 12, further comprising a gas-capturing portion configured to capture an inert gas and carbon dioxide from air or a biogas,
wherein a gas containing the inert gas and the carbon dioxide, which is to be supplied from the gas-capturing portion, is supplied as a constituent component for the raw material gas to the reactor.

16. The liquid fuel synthesis system according to any one of claims 12 to 15, wherein the reactor includes a water vapor separation membrane configured to cause water vapor to permeate therethrough.

17. The liquid fuel synthesis system according to any one of claims 12 to 15, wherein the reactor includes a liquid fuel separation membrane configured to cause at least the liquid fuel to permeate therethrough.
